(19) Europäisches Patentamt
European Patent Office
Office européen des brevets

(11) Veröffentlichungsnummer: **0 186 027**
**A2**

(12) # EUROPÄISCHE PATENTANMELDUNG

(21) Anmeldenummer: **85115636.4**

(22) Anmeldetag: **09.12.85**

(51) Int. Cl.[4]: **A 61 K 31/44**

(30) Priorität: **22.12.84 DE 3447170**

(43) Veröffentlichungstag der Anmeldung:
**02.07.86 Patentblatt 86/27**

(84) Benannte Vertragsstaaten:
**AT BE CH DE FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Goldmann, Siegfried, Dr.**
**Am Osterholz 91**
**D-5600 Wuppertal 11(DE)**

(72) Erfinder: **Böshagen, Horst, Dr.**
**Wiesenstrasse 4**
**D-5657 Haan(DE)**

(72) Erfinder: **Bossert, Friedrich, Dr.**
**Claudiusweg 7**
**D-5600 Wuppertal 1(DE)**

(72) Erfinder: **Franckowiak, Gerhard, Dr.**
**Henselweg 10**
**D-5600 Wuppertal 1(DE)**

(72) Erfinder: **Meyer, Horst, Dr.**
**Henselweg 11**
**D-5600 Wuppertal 1(DE)**

(72) Erfinder: **Stoltefuss, Jürgen, Dipl.-Ing.**
**Parkstrasse 20**
**D-5657 Haan(DE)**

(72) Erfinder: **Gross, Rainer, Dr.**
**Platzhofstrasse 23**
**D-5600 Wuppertal 1(DE)**

(72) Erfinder: **Schramm, Mathias, Dr.**
**Paffrather Strasse 38**
**D-5000 Koeln 80(DE)**

(72) Erfinder: **Thomas, Günter, Dr.**
**Henselweg 5**
**D-5600 Wuppertal 1(DE)**

(54) **Mischung unterschiedlicher Dihydropyridine, Verfahren zu ihrer Herstellung sowie ihre Verwendung in Arzneimitteln.**

(57) Die Erfindung betrifft eine Mischung enthaltend
a) Dihydropyridine der allgemeinen Formel (I)

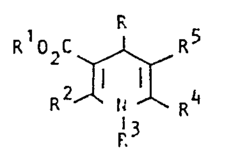

(I)

und

b) Dihydropyridine der allgemeinen Formel (II)

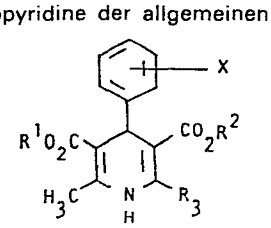

(II)

mit in den Beschreibung näher definierten Substituenten, ein Verfahren zu ihrer Herstellung sowie ihre Verwendung in Arzneimitteln.

EP 0 186 027 A2

Croydon Printing Company Ltd

0186027

BAYER AKTIENGESELLSCHAFT 5090 Leverkusen, Bayerwerk
Konzernverwaltung RP E/by-c
Patentabteilung

# Mischung unterschiedlicher Dihydropyridine, Verfahren zu ihrer Herstellung sowie ihre Verwendung in Arzneimitteln

Die Erfindung betrifft eine Wirkstoffkombination mit positiv inotroper und antianginöser Wirkung enthaltend positiv inotrope Dihydropyridine (Komponente A) und vasodilatierende Dihydropyridine (Komponente B), Verfahren zu ihrer Herstellung sowie ihre Verwendung in Arzneimitteln.

Als positiv inotrope Dihydropyridine (Komponente A) seien diejenigen der folgenden Formel (I) genannt

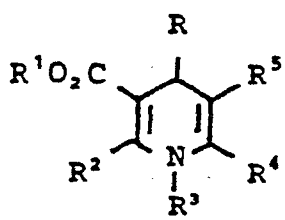

(I)

in welcher

R -für Cycloalkyl ($C_3$-$C_{14}$) steht, oder
-für Aryl ($C_6$-$C_{14}$) oder Heteroaryl steht, die gegebenenfalls substituiert sind durch bis zu fünf gleiche oder verschiedene Substituenten der Reihe: Halogen, Nitro, Cyano, Trifluormethyl, Monofluoralkoxy ($C_1$-$C_{12}$), Polyfluoralkoxy ($C_1$-$C_{12}$), Hydroxy, Amino, Alkylamino, Dialkylamino (Alkyl jeweils $C_1$-$C_8$), Aryl ($C_6$-$C_{14}$), Heteroaryl, Aralkyl ($C_7$-$C_{14}$), -O-Aralkyl ($C_7$-$C_{14}$) oder -$SO_n$-Aralkyl ($C_7$-$C_{14}$; n = 0-2), wobei Substituenten der fünf letztgenannten Gruppen ebenfalls mehrfach substituiert sein können durch bis zu fünf Substituenten der Reihe: Halogen, Nitro, Azido, Hydroxy, Trifluormethyl, Trifluormethoxy, Cyano, Amino, Alkylamino, Dialkylamino (Alkyl jeweils $C_1$-$C_8$), Alkoxy, Alkylthio (Alkyl jeweils $C_1$-$C_4$),

$R^1$ - für einen geradkettigen, verzweigten oder cyclischen, gesättigten oder ungesättigten Kohlenwasserstoffrest (bis $C_{20}$) steht, der gegebenenfalls durch bis zu fünf Schwefel- und/oder Sauerstoffatome in der Kette unterbrochen ist und der gegebenenfalls substituiert ist durch Halogen, Nitro, Hydroxy, Cyano, Trialkylsilyl (Alkyl jeweils $C_1$-$C_8$), Alkoxycarbonyl ($C_1$-$C_4$), Amino, Alkylamino oder Dialkylamino (Alkyl jeweils $C_1$-$C_4$),

$R^2$, $R^4$ - gleich oder verschieden sind und
- für Wasserstoff,
- für Amino,
- für Cyano,
- für Formyl oder

- **für geradkettiges, verzweigtes oder cyclisches**, gegebenenfalls durch Hydroxy, Carboxy, Alkoxycarbonyl ($C_1$-$C_4$), oder Halogen substituiertes Alkyl oder Alkenyl (bis $C_{10}$) stehen,

$R^3$ - **für Wasserstoff oder**

- **für geradkettiges, verzweigtes oder** cyclisches Alkyl oder Alkenyl (bis $C_{10}$) steht, das gegebenenfalls substituiert ist durch Halogen, Cyano, Hydroxy, Amino, Alkylamino, Dialkylamino **(Alkyl jeweils $C_1$-$C_4$) oder durch eine 5-7 gliedrigen heterocyclischen Ring der als Heteroatome Stickstoff und/oder Sauerstoff und/oder Schwefel enthalten kann und sowohl gesättigt als auch ungesättigt sein kann,**

**und**

$R^5$ - **für Wasserstoff,**

- **für** Cyano,
- **für Nitro steht,**

**oder**

$R^4$ und $R^5$ **gemeinsam einen Ring bilden wie**

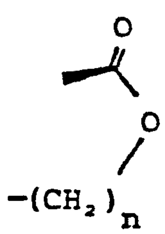

,

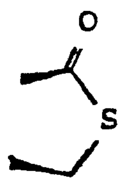

oder

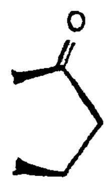

wobei n für 1 oder 2 steht.

Bevorzugte Verbindungen der allgemeinen Formel (I) sind solche, in welchen

R - für Cycloalkyl ($C_4$-$C_{12}$) steht, oder
- für Phenyl, Naphthyl, Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Chinolyl, Isochinolyl, Indolyl, Benzimidazolyl, Chinazolyl, Chinoxalyl, Thionaphthenyl, Isothionaphthenyl, Chromonyl, Thiochromonyl, Chromenyl, Thiochromenyl, Benzoxadiazolyl oder Benzthiadiazolyl steht, wobei die genannten Reste gegebenenfalls substituiert sind durch bis zu vier gleiche oder verschiedene Substituenten aus der Reihe: Fluor, Chlor, Brom, Jod, Nitro, Cyano, Trifluormethyl, Monofluoralkoxy ($C_1$-$C_8$), Polyfluoralkoxy ($C_1$-$C_8$), Hydroxy, Amino, Alkylamino, Dialkylamino (Alkyl jeweils $C_1$-$C_6$), Phenyl, Naphthyl, Thienyl, Furyl, Pyridyl, Pyrimidyl, Benzyl, -O-Benzyl, -$SO_n$-Benzyl (n = 0-2) wobei die Aromaten und Heteroaromaten gegebenenfalls wiederum ein- bis vierfach substituiert sein können durch Fluor, Chlor, Brom, Jod, Cyano, Nitro, Azido, Hydroxy, Trifluormethyl, Trifluormethoxy, Amino, Alkylamino, Dialkylamino (Alkyl jeweils $C_1$-$C_6$), Alkoxy oder Alkylthio (jeweils $C_1$-$C_2$),

$R^1$ - für einen geradkettigen, verzweigten oder cyclisch gesättigten oder ungesättigten Kohlenwasserstoffrest (bis $C_{17}$) steht, der gegebenenfalls in der Kette durch bis zu vier Sauerstoff- und/oder Schwefelatome unterbrochen ist und der gegebenenfalls substituiert ist durch ein oder mehrere Fluor, Chlor, Brom, Jod, Nitro, Hydroxy, Cyano, Trialkylsilyl ($C_1$-$C_6$), Alkoxycarbonyl ($C_1$-$C_2$), Amino, Alkylamino oder Dialkylamino (Alkyl jeweils $C_1$-$C_2$),

$R^2$,$R^4$ - gleich oder verschieden sind und
- für Wasserstoff,
- für Amino,
- für Cyano,
- für Formyl oder
- für geradkettiges, verzweigtes oder cyclisches, gegebenenfalls durch Hydroxy, Carboxy, Alkoxycarbonyl ($C_1$-$C_2$), ein oder mehrere Fluor, Chlor oder Brom substituiertes Alkyl oder Alkenyl (bis $C_8$) stehen,

$R^3$- für Wasserstoff oder
- für geradkettiges, verzweigtes oder cyclisches Alkyl oder Alkenyl (bis $C_8$) steht, das gegebenen- das gegebenenfalls durch bis zu 3 Sauerstoffatome in der Kette unterbrochen ist und das gegebenenfalls substituiert ist durch ein oder mehrere Fluor, Chlor, Brom, Jod, Cyano, Hydroxy, Amino, Alkylamino, Dialkylamino (Alkyl jeweils $C_1$-$C_2$), Morpholin, Piperidin, Pyridazin oder Pyridin,

und

$R^5$ - für Wasserstoff,
- für Cyano,
- für Nitro steht,

oder

$R^4$ und $R^5$ gemeinsam einen Ring bilden wie

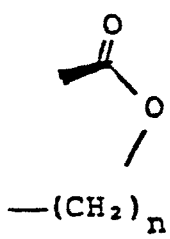 , 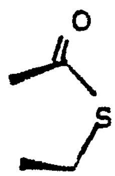 oder 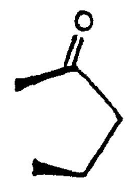

wobei n für 1 oder 2 steht.

Besonders bevorzugt sind Verbindungen der Formel (I), in welcher

R — für Cycloalkyl ($C_5$-$C_{10}$) steht,
— für Phenyl, Naphthyl, Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Chinazolyl, Chinoxalyl, Thionaphthenyl, Isothionaphthenyl, Chromonyl, Thiochromonyl, Chromenyl, Thiochromenyl, Benzoxadiazolyl oder Benzthiadiazolyl steht, wobei

die genannten Reste gegebenenfalls substituiert sind durch bis zu drei gleiche oder verschiedene Substituenten der Reihe: Fluor, Chlor, Brom, Nitro, Cyano, Trifluormethyl, Monofluoralkoxy ($C_1$-$C_4$), Polyfluoralkoxy ($C_1$-$C_4$), Hydroxy, Amino, Alkylamino, Dialkylamino (Alkyl jeweils $C_1$-$C_4$), Phenyl, Thienyl, Pyridyl, Benzyl, -O-Benzyl, -$SO_n$-Benzyl (n = 0 bis 2), wobei die Aromaten und Heteroaromaten gegebenenfalls wiederum ein- bis dreifach substituiert sein können durch Fluor, Chlor, Brom, Cyano, Nitro, Hydroxy, Trifluormethyl, Trifluormethoxy, Amino, Alkylamino, Dialkylamino (Alkyl jeweils $C_1$-$C_4$), Methoxy oder Methylthio,

$R^1$ - für einen geradkettigen, verzweigten oder cyclischen, gesättigten oder ungesättigten Kohlenwasserstoffrest (bis $C_{14}$) steht, der gegebenenfalls durch bis zu drei Sauerstoff- und/oder Schwefelatome in der Kette unterbrochen sein kann und der gegebenenfalls substituiert ist durch ein oder mehrere Fluor, Chlor, Brom, Nitro, Hydroxy, Cyano oder Trialkylsilyl ($C_1$-$C_4$),

$R^2$,$R^4$ - gleich oder verschieden sind und
- für Wasserstoff
- für Amino
- für Cyano
- für Formyl oder

- für geradkettiges oder verzweigtes, gegebenenfalls durch Hydroxy substituiertes Alkyl oder Alkenyl (bis $C_6$) stehen,

$R^3$ - für Wasserstoff oder
- für geradkettiges, verzweigtes oder cyclisches, Alkyl oder Alkenyl (bis $C_6$) steht, das gegebenenfalls durch bis zu 2 Sauerstoffatome in der Kette unterbrochen ist und das gegebenenfalls substituiert ist durch ein oder mehrere Fluor, Chlor, Cyano, Hydroxy, Amino oder Morpholino,

und

$R^5$ - für Wasserstoff,
- für Cyano,
- für Nitro steht,

oder

$R^4$ und $R^5$ gemeinsam einen Ring bilden wie:

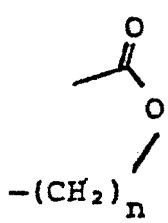

,

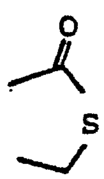

oder

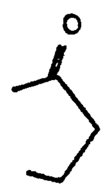

wobei n für 1 oder 2 steht.

Insbesondere seien genannt:
1,4-Dihydro-2,6-dimethyl-5-nitro-4-(2-trifluormethylphenyl)-pyridin-3-carbonsäuremethylester (BAY K 8644)
2-Methyl-4-(4-oxo-2-phenyl-4H-thiochromen-8-yl)-5-oxo-1,4,5,7-tetrahydrofuro/3̅,4-b̲7pyridin-3-carbonsäureethylester.

Die Herstellung dieser Verbindungen ist in den Deutschen Offenlegungsschriften 32 06 671, 33 11 005 und 33 11 003 beschrieben.

Als vasodilatierende Dihydropyridine (Komponente B) seien diejenigen der folgenden Formel (II) genannt,

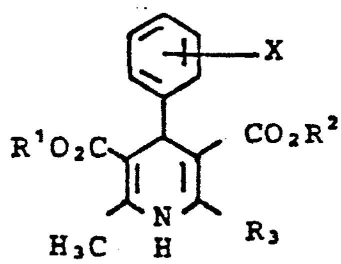

(II)

in welcher

$R^1$ - für gegebenenfalls durch Alkoxy ($C_1$-$C_3$) substituiertes Alkyl ($C_1$-$C_4$) steht,

$R^2$ - für Alkyl ($C_1$-$C_{10}$) steht, das gegebenenfalls substituiert ist durch Alkoxy ($C_1$-$C_3$), Trifluormethyl, Trifluorethyl, N-Methyl-N-benzylamino,

$R^3$ - für Cyano,
- für Hydroxymethyl oder
- für Alkyl ($C_1$-$C_4$) steht,

und

X - für 2-Nitro, 3-Nitro, 2-Chlor, 2,3-Dichlor, 2,3-Ringglied bestehend aus =N-O-N= steht.

Besonders bevorzugt sind die Verbindungen der folgenden Tabelle:

Tabelle

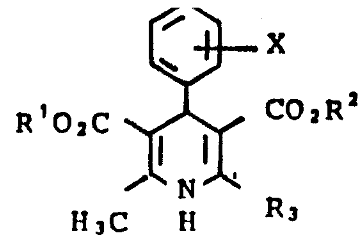

| Nr. | X | $R^1$ | $R^2$ | $R^3$ | Generic |
|---|---|---|---|---|---|
| 1 | $2-NO_2$ | $CH_3$ | $CH_3$ | $CH_3$ | Nifedipin |
| 2 | $3-NO_2$ | $nPrOCH_2CH_2$ | $nPrOCH_2CH_2$ | $CH_3$ | Niludipin |
| 3 | $3-NO_2$ | $C_2H_5$ | $CH_3$ | $CH_3$ | Nitrendipin |
| 4 | $2-NO_2$ | $CH_3$ | $(CH_3)_2CHCH_2$ | $CH_3$ | Nisoldipin |
| 5 | $3-NO_2$ | $CH(CH_3)_2$ | $(CH_2)_2-O-CH_3$ | $CH_3$ | Nimodipin |
| 6 | $3-NO_2$ | $C_2H_5$ | $C_{10}H_{21}(n)$ | $CH_3$ | |
| 7 | 2-Cl | $CH_3$ | $CH_2-CF_3$ | $CH_3$ | |
| 8 | 2-Cl | $C_2H_5$ | $CH_2-CF_3$ | $CH_3$ | |
| 9 | $3-NO_2$ | $CH(CH_3)_2$ | $n-PrO-CH_2CH_2$ | $CH_3$ | |
| 10 | $3-NO_2$ | $CH_3$ | $C_6H_5CH_2N(CH_3)CH_2CH_2$ | $CH_3$ | Nicardipin |
| 11 | $2,3-Cl_2$ | $C_2H_5$ | $CH_3$ | $CH_3$ | Felodipin |
| 12 | 2,3=N-O-N= | $C_2H_5$ | $C_2H_5$ | $CH_3$ | Dazodipin |
| 13 | 2,3=N-O-N= | $CH_3$ | $CH(CH_3)_2$ | $CH_3$ | (PN 200-110) |
| 14 | $3-NO_2$ | $C_2H_5$ | $C_2H_5$ | $CH_2OH$ | |
| 15 | $3-NO_2$ | $CH_3$ | $CH_3$ | CN | Nivadipin |

n-Pr =n-Propyl

Insbesondere seien genannt Nitrendipin, Nicardipin, Nisoldipin, Felodipin, Nifedipin, Nimodipin, Produkt Tabelle Nr. 13, Dazodipin. Die Herstellung dieser Verbindungen ist beschrieben in US 34 85 847, EP 7293, DOS 24 07 115, DOS 25 49 568, DOS 21 17 571, DOS 29 49 464, DOS 29 49 491.

Da sowohl calciumantagonistische als auch positiv inotrope Dihydropyridine an den gleichen Rezeptor binden, würde man für die Kombination beider Komponenten nur eine Abschwächung der Einzelwirkung erwarten.

Jedoch zeigt die Kombination bei geeigneten Verhältnissen der Komponenten A und B ein völlig überraschendes Wirkprofil: Sie ist positiv inotrop und gefäßdilatierend, insbesondere coronardilatierend.

In die Mischung können 1 bis 10 Gewichtsteile der Komponente A und 0,1 bis 100 Gewichtsteile, bevorzugt 0,1 bis 10 Gewichtsteile der Komponente B eingesetzt werden.

Die Erfindung betrifft außerdem Kombinationen von positiv inotropen, racemischen Dihydropyridinen mit vasodilatierenden, optisch aktiven Dihydropyridinen, Kombinationen von vasodilatierenden, racemischen Dihydropyridinen mit positiv inotropen, optisch aktiven Dihydropyridinen sowie Kombinationen von optisch aktiven, positiv inotropen Dihydropyridinen mit optisch aktiven, vasodilatierenden Dihydropyridinen.

Die Kombination kann hergestellt werden, indem man die Einzelkomponenten in diese auflösenden inerten Lösungsmittel auflöst und diese Lösungen in den entsprechenden Mengenverhältnissen mischt.

Als inerte Lösungsmittel seien beispielhaft Alkohole wie Ethanol oder Glykole wie Polyethylenglykol, insbesondere Dimethylsulfoxid genannt.

Wie bereits erwähnt, kann die erfindungsgemäße Kombination zur Bekämpfung von Krankheiten, insbesondere von Herz-Kreislauferkrankungen wie Bluthochdruck, ischämischen Herzerkrankungen oder Herzversagen eingesetzt werden.

Die Wirkstoffkombination kann in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter nicht toxischer pharmazeutisch geeigneter Trägerstoffe oder Lösungsmittel. Hierbei sollen die therapeutisch wirksamen Verbindungen jeweils in einer Konzentration von etwa 0,01 bis 90 Gew.-% der Gesamtmischung vorhanden sein, d.h. in Mengen, die ausreichend sind, um den angegebenen Dosierungsspielraum zu erreichen.

Diese Formulierungen werden beispielsweise hergestellt durch Verstrecken der Wirkstoffkombination mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:

Wasser, nicht-toxische organische Lösungsmittel, wie Paraffine (z.B. Erdölfraktionen), pflanzliche Öle (z.B. Erdnuß-/Sesam-Öl), Alkohole (z.B. Ethylalkohol, Glycerin), Glykole (z.B. Propylenglykol, Polyethylenglykol), feste Trägerstoffe wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether, Alkylsulfonate und Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet

werden. Im Falle wäßriger Suspensionen und/oder Elixieren, die für orale Anwendungen gedacht sind, kann die Wirkstoffkombination außer mit den obengenannten Hilfsstoffen mit verschiedenen Geschmacksaufbesserern oder Farbstoffen versetzt werden.

Für den Fall der parenteralen Anwendung können Lösungen der Wirkstoffkombination unter Verwendung geeigneter flüssiger Trägermaterialien eingesetzt werden.

Die Herz- und Gefäßwirkungen der erfindungsgemäßen Kombinationen wurden an isoliert perfundierten Herzen des Meerschweinchens gefunden (modifiziert nach Opie, L., J. Physiol. 180 (1965) 529-541). Dazu werden die Herzen von 250 bis 350 g schweren Albino Meerschweinchen verwendet. Die Tiere werden mit einem Schlag auf den Kopf getötet, der Thorax geöffnet, in die freipräparierte Aorta eine Metallkanüle eingebunden und der linke Vorhof eröffnet. Das Herz wird mit den Lungen aus dem Thorax herausgetrennt und über die Aortenkanüle an die Perfusionsapparatur bei laufender Perfusion angeschlossen. Die Lungen werden an den Lungenwurzeln abgetrennt. Als Perfusionsmedium dient Krebs-Henseleit-Lösung (118,5 mmol/l NaCl, 4,75 mmol/l KCl, 1,19 mmol/l $MgSO_4$, 25 mmol/l $NaHCO_3$, 0,013 mmol/l NaEDTA, deren $CaCl_2$-Konzentration je nach Bedarf variiert wird, in der Regel jedoch 1,2 mmol/l beträgt. Als energieliefernes Substrat werden 10 mmol/l Glucose zugesetzt. Vor der Perfusion wird die Lösung partikelfrei filtriert. Die Lösung wird mit Carbogen (95 % $O_2$, 5 % $CO_2$ zur Aufrechterhaltung des pH-Wertes von 7,4) begast.

Die Herzen werden mit konstantem Fluß (10 ml/min) bei 32°C mittels einer Rollenquetschpumpe perfundiert.

Zur Messung der Herzfunktion wird ein flüssigkeitsgefüllter Latexballon, der über eine Flüssigkeitssäule mit einem Druckaufnehmer verbunden ist, durch den linken Vorhof in den linken Ventrikel eingeführt und die isovolumetrischen Kontraktionen auf einem Schnellschreiber registriert.

Der Perfusionsdruck als Maß des Coronarwiderstandes wird mittels eines Druckaufnehmers registriert. Unter diesen Bedingungen zeigt eine Senkung des Perfusionsdruckes eine Koronardilatation, eine Steigerung der linksventrikulären Druckamplitude einen Anstieg der Herzkontraktilität an. Die erfindungsgemäßen Kombinationen werden in geeigneten Verdünnungen in das Perfusionssystem kurz vor dem isolierten Herzen infundiert.

So wirken die in der folgenden Tabelle beispielhaft aufgeführten Kombinationen am isoliert perfundierten Meerschweinchenherzen positiv inotrop und coronardilatierend.

| Kombination | | KA | PD |
|---|---|---|---|
| Komponente A | Komponente B | (Prozentualer Anstieg (+) oder Abfall (-) im Vergleich zur Kontrolle) | |
| Aus Kombination 1 | | | |
| BAY K 8644 : | Nifedipin | + 38 | -18 |
| (100 nmol/l) | (100 nmol/l) | | |
| Aus Kombination 2 | | | |
| BAY K 8644 : | Nisoldipin | + 32 | -21 |
| (100 nmol/l) | (100 nmol/l) | | |
| Aus Kombination 3 | | | |
| BAY K 8644 : | Nicardipin | + 18 | -17 |
| (100 nmol/l) | (200 nmol/l) | | |
| Aus Kombination 4 | | | |
| BAY K 8644 : | Nitrendipin | + 47 | -24 |
| (30 nmol/l) | (90 nmol/l) | | |

KA = Kontraktionsamplitude

PD = Perfusionsdruck

Die Kombinationen 1 bis 4 werden folgendermaßen hergestellt:

Kombination 1

Zunächst werden $3{,}6 \cdot 10^{-3}$ g BAY K 8644 in einem Milliliter DMSO gelöst, dann werden $3{,}5 \cdot 10^{-3}$ g Nifedipin in einem Milliliter DMSO gelöst und anschließend die beiden Lösungen im Verhältnis 1:1 gemischt.

Kombination 2

Zunächst werden $3{,}6 \cdot 10^{-3}$ g BAY K 8644 in einem Milliliter DMSO gelöst, dann werden $3{,}9 \cdot 10^{-3}$ g Nisoldipin in einem Milliliter DMSO gelöst und anschließend die beiden Lösungen im Verhältnis 1:1 gemischt.

Kombination 3

Zunächst werden $3{,}6 \cdot 10^{-3}$ g BAY K 8644 in einem Milliliter DMSO gelöst, dann werden $9{,}3 \cdot 10^{-3}$ g Nicardipin in einem Milliliter DMSO gelöst und anschließend die beiden Lösungen im Verhältnis 1:1 gemischt.

Kombination 4

Zunächst werden $1 \cdot 10^{-3}$ g BAY K 8644 in einem Milliliter DMSO gelöst, dann werden $3{,}2 \cdot 10^{-3}$ g Nitrendipin in einem Milliliter DMSO gelöst und anschließend die beiden Lösungen im Verhältnis 1:1 gemischt.

Jeweils anschließend wird eine Infusionslösung enthaltend 0,9 % NaCl aus der Kombination hergestellt.

0186027

Patentansprüche

1. Mischung enthaltend

a) Dihydropyridine der allgemeinen Formel (I)

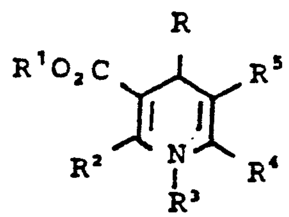

(I)

in welcher

R - für Cycloalkyl steht, oder
- Aryl oder Heteroaryl steht, die gegebenenfalls substituiert sind durch bis zu fünf gleiche oder verschiedene Substituenten der Reihe: Halogen, Nitro, Cyano, Trifluormethyl, Monofluoralkoxy, Polyfluoralkoxy, Hydroxy, Amino, Alkylamino, Dialkylamino, Aryl, Heteroaryl, Aralkyl, -O-Aralkyl, oder $-SO_n$-Aralkyl (n = 0-2), wobei Substituenten der fünf letztgenannten Gruppen ebenfalls mehrfach substituiert sein können durch bis zu fünf Substituenten der Reihe: Halogen, Nitro, Azido, Hydroxy, Trifluormethyl, Trifluormethoxy, Cyano, Amino, Alkylamino, Dialkylamino, Alkoxy, Alkylthio,

$R^1$ - für einen geradkettigen, verzweigten oder cyclischen, gesättigten oder ungesättigten Kohlenwasserstoffrest steht, der gegebenenfalls durch

bis zu fünf Schwefel- und/oder Sauerstoffatome in der Kette unterbrochen ist und der gegebenenfalls substituiert ist durch Halogen, Nitro, Hydroxy, Cyano, Trialkylsilyl, Alkoxycarbonyl, Amino, Alkylamino oder Dialkylamino,

$R^2$, $R^4$ - gleich oder verschieden sind und
- für Wasserstoff,
- für Amino,
- für Cyano,
- für Formyl oder
- für geradkettiges, verzweigtes oder cyclisches gesättigtes oder ungesättigtes, gegebenenfalls durch Hydroxy, Carboxy, Alkoxycarbonyl oder Halogen substituiertes Alkyl,

$R^3$ - für Wasserstoff oder
- für geradkettiges, verzweigtes oder cyclisches Alkyl oder Alkenyl steht,
das gegebenenfalls substituiert ist durch Halogen, Cyano, Hydroxy, Amino, Alkylamino, Dialkylamino oder durch eine 5-7 gliedrigen heterocyclischen Ring der als Heteroatome Stickstoff und/oder Sauerstoff und/oder Schwefel enthalten kann und sowohl gesättigt als auch ungesättigt sein kann,

und

$R^5$ - für Wasserstoff,
- für Cyano,
- für Nitro steht,

oder

$R^4$ und $R^5$ gemeinsam einen Ring bilden wie

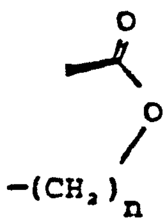 , 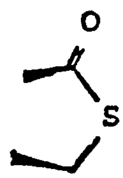 oder 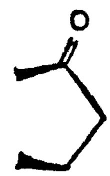

wobei n für 1 oder 2 steht,

und

b) Dihydropyridine der allgemeinen Formel (II)

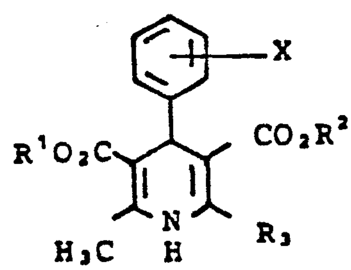

(II)

in welcher

$R^1$ - für gegebenenfalls durch $C_1$-$C_3$ Alkoxy substituiertes $C_1$-$C_4$ Alkyl steht,

$R^2$ - für $C_1$-$C_{10}$ Alkyl steht, das gegebenenfalls substituiert ist durch $C_1$-$C_3$ Alkoxy, Trifluormethyl, Trifluorethyl, N-Methyl-N-benzylamino,

$R^3$ - für Cyano,
- für Hydroxymethyl oder
- für $C_1$-$C_4$ Alkyl steht,

und

X - für 2-Nitro, 3-Nitro, 2-Chlor, 2,3-Dichlor, 2,3-Ringglied bestehend aus =N-O-N= steht.

2. Mischung gemäß Anspruch 1 enthaltend als Komponente a) Dihydropyridine der allgemeinen Formel (I) in Anspruch 1, in welcher

R - für $C_4$-$C_{12}$ Cycloalkyl steht, oder
- für Phenyl, Naphthyl, Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Chinolyl, Isochinolyl, Indolyl, Benzimidazolyl, Chinazolyl, Chinoxalyl, Thionaphthenyl, Isothionaphthenyl, Chromonyl, Thiochromonyl, Chromenyl, Thiochromenyl, Benzoxadiazolyl oder Benzthiadiazolyl steht, wobei die genannten Reste gegebenenfalls substituiert sind durch bis zu vier gleiche oder verschiedene Substituenten aus der Reihe: Fluor, Chlor, Brom, Jod, Nitro, Cyan, Trifluormethyl, Mono-

fluor-$C_1$-$C_8$-alkoxy, Polyfluor-$C_1$-$C_8$-alkoxy, Hydroxy, Amino, Alkylamino, Di-$C_1$-$C_6$-alkylamino, Phenyl, Naphthyl, Thienyl, Furyl, Pyridyl, Pyrimidyl, Benzyl, -O-Benzyl, -$SO_n$-Benzyl (n = 0-2) wobei die Aromaten und Heteroaromaten gegebenenfalls wiederum ein- bis vierfach substituiert sein können durch Fluor, Chlor, Brom, Jod, Cyano, Nitro, Azido, Hydroxy, Trifluormethyl, Trifluormethoxy, Amino, Alkylamino, Di-$C_1$-$C_6$-alkylamino, Alkoxy oder $C_1$-$C_2$-Alkylthio,

$R^1$ - für einen geradkettigen, verzweigten oder cyclisch gesättigten oder ungesättigten Kohlenwasserstoffrest mit bis zu 17 C-Atomen steht, der gegebenenfalls in der Kette durch bis zu vier Sauerstoff- und/oder Schwefelatome unterbrochen ist und der gegebenenfalls substituiert ist durch ein oder mehrere Fluor, Chlor, Brom, Jod, Nitro, Hydroxy, Cyano, Tri-$C_1$-$C_6$-alkylsilyl, $C_1$-$C_2$-Alkoxycarbonyl, Amino, Alkylamino oder Di-$C_1$-$C_2$-alkylamino,

$R^2$,$R^4$ - gleich oder verschieden sind und
- für Wasserstoff,
- für Amino,
- für Cyano,
- für Formyl oder
- für geradkettiges, verzweigtes oder cyclisches, gegebenenfalls

durch Hydroxy, Carboxy, $C_1$-$C_2$-Alkoxycarbonyl, ein oder mehrere Fluor, Chlor oder Brom substituiertes $C_1$-$C_8$-Alkyl oder -Alkenyl stehen,

$R^3$ - für Wasserstoff oder
- für geradkettiges, verzweigtes oder cyclisches, $C_1$-$C_8$-Alkyl oder -Alkenyl steht, das gegebenenfalls durch bis zu 3 Sauerstoffatome in der Kette unterbrochen ist und das gegebenenfalls substituiert ist durch ein oder mehrere Fluor, Chlor, Brom, Jod, Cyano, Hydroxy, Amino, $C_1$-$C_2$-Alkyl, Di-$C_1$-$C_2$-alkylamino, Morpholin, Piperidin, Pyridazin oder Pyridin,

und

$R^5$ - für Wasserstoff,
- für Cyano,
- für Nitro steht,

oder

$R^4$ und $R^5$ gemeinsam einen Ring bilden wie

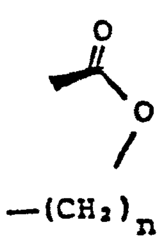 , 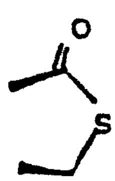 oder 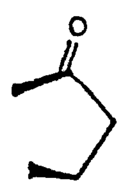

wobei n für 1 oder 2 steht.

3. Mischung gemäß Anspruch 1 enthaltend als Komponente a) Dihydropyridin der allgemeinen Formel (I) in Anspruch 1, in welcher

R - für $C_5$-$C_{10}$-Cycloalkyl steht,
- für Phenyl, Naphthyl, Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Chinazolyl, Chinoxalyl, Thionaphthenyl, Isothionaphthenyl, Chromoyl, Thiochromonyl, Chromenyl, Thiochromenyl, Benzoxadiazolyl oder Benzthiadiazolyl steht, wobei die genannten Reste gegebenenfalls substituiert sind durch bis zu drei gleiche oder verschiedene Substituenten der Reihe: Fluor, Chlor, Brom, Nitro, Cyano, Trifluormethyl, Monofluor-$C_1$-$C_4$-alkoxy, Polyfluor-$C_1$-$C_4$-alkoxy, Hydroxy, Amino, $C_1$-$C_4$-Alkylamino, Di-$C_1$-$C_4$-alkylamino, Phenyl, Thienyl, Pyridyl, Benzyl, -O-Benzyl, -$SO_n$-Benzyl (n = 0 bis 2), wobei die Aromaten und Heteroaromaten gegebenenfalls wiederum ein- bis dreifach substituiert sein können durch Fluor, Chlor, Brom, Cyano, Nitro, Hydroxy, Trifluormethyl, Trifluormethoxy, Amino, $C_1$-$C_4$-Alkylamino, Di-$C_1$-$C_4$-alkylamino, Methoxy oder Methylthio,

$R^1$ - für einen geradkettigen, verzweigten oder cyclischen, gesättigten oder ungesättigten Kohlenwasserstoffrest mit bis zu 14 C-Atomen

steht, der gegebenenfalls durch bis zu 3 Sauerstoff- und/oder Schwefelatome in der Kette unterbrochen sein kann und der gegebenenfalls substituiert ist durch ein oder mehrere Fluor, Chlor, Brom, Nitro, Hydroxy, Cyano, oder Tri-$C_1$-$C_4$-alkylsilyl,

$R^2$,$R^4$ - gleich oder verschieden sind und
- für Wasserstoff,
- für Amino,
- für Cyano,
- für Formyl oder
- für geradkettiges oder verzweigtes, gegebenenfalls durch Hydroxy substituiertes $C_1$-$C_4$-Alkyl oder -Alkenyl stehen,

$R^3$ - für Wasserstoff oder
- für geradkettiges, verzweigtes oder cyclisches $C_1$-$C_6$-Alkyl oder -Alkenyl steht, das gegebenenfalls durch bis zu zwei Sauerstoffatome in der Kette unterbrochen ist und das gegebenenfalls substituiert ist durch ein oder mehrere Fluor, Chlor, Cyan, Hydroxy, Amino oder Morpholino,

und

$R^5$ - für Wasserstoff,
- für Cyano,
- für Nitro steht,

oder

$R^4$ und $R^5$ gemeinsam einen Ring bilden wie

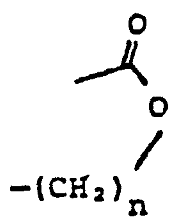
,
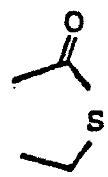
oder
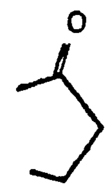

wobei n für 1 oder 2 steht.

4. Mischung gemäß Anspruch 1 enthaltend als Komponente a) 1,4-Dihydro-2,6-dimethyl-5-nitro-4-(2-trifluormethylphenyl)-pyridin-3-carbonsäuremethylester und/oder 2-Methyl-4-(4-oxo-2-phenyl-4H-thiochromen-8-yl)-5-oxo-1,4,5,7-tetrahydrofuro[3,4-b]pyridin-3-carbonsäureethylester.

5. Mischung gemäß Ansprüchen 1 - 4 enthaltend als Komponente b) ein Dihydropyridin der Formel (II) in Anspruch 1, in welcher

X eine Nitrogruppe in 2- bzw. 3-Position, 2-Chlor, 2,3-Dichlor, oder eine 2,3 =N-O-N= Gruppe darstellt,

$R^1$ Methyl, Ethyl, Isopropyl, n-Propyloxyethyl darstellt,

$R^2$ Methyl, Ethyl, Isopropyl, Isobutyl, n-Decyl, n-Propyloxyethyl, Methoxyethyl, Trifluormethylmethyl sowie die Gruppe $C_6H_5CH_2N(CH_3)-CH_2-CH_2-$ darstellt und

$R^3$ Methyl, Cyano und Hydroxymethyl darstellt.

6. Mischung gemäß Ansprüche 1 bis 4 enthaltend als Komponente b) Nitrendipin, Nicardipin, Nisoldipin, Felodipin, Nifedipin, Nimodipin, PN 200-100, Dazodipin.

7. Mischung gemäß Ansprüche 1 bis 6 enthaltend 1 bis 10 Gewichtsteile der Komponente a) und 0,1 bis 100 Gewichtsteile der Komponente b).

8. Mischung gemäß Ansprüche 1 bis 7 zur Bekämpfung von Erkrankungen.

9. Arzneimittel enthaltend als Wirkstoffkombination die Mischung gemäß Ansprüche 1 bis 7.

10. Verfahren zur Herstellung der Mischung aus

a) Dihydropyridinen der allgemeinen Formel (I)

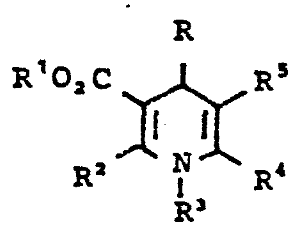

(I)

in welcher

R - für Cycloalkyl steht, oder
- Aryl oder Heteroaryl steht, die gegebenenfalls substituiert sind durch bis zu fünf gleiche oder verschiedene Substituenten der Reihe: Halogen, Nitro, Cyano, Trifluormethyl, Monofluoralkoxy, Polyfluoralkoxy, Hydroxy, Amino, Alkylamino, Dialkylamino, Aryl, Heteroaryl, Aralkyl, -O-Aralkyl, oder $-SO_n$-Aralkyl (n = 0-2), wobei Substituenten der fünf letztgenannten Gruppen ebenfalls mehrfach substituiert sein können durch bis zu fünf Substituenten der Reihe: Halogen, Nitro, Azido, Hydroxy, Trifluormethyl, Trifluormethoxy, Cyano, Amino, Alkylamino, Dialkylamino, Alkoxy, Alkylthio,

$R^1$ - für einen geradkettigen, verzweigten oder cyclischen, gesättigten oder ungesättigten Kohlenwasserstoffrest steht, der gegebenenfalls durch bis zu fünf Schwefel- und/oder Sauerstoffatome in der Kette unterbrochen ist und der gegebenenfalls substituiert ist durch Halogen, Nitro, Hydroxy, Cyano, Trialkylsilyl, Alkoxycarbonyl, Amino, Alkylamino oder Dialkylamino,

$R^2$, $R^4$ - gleich oder verschieden sind und
- für Wasserstoff,
- für Amino,
- für Cyano,
- für Formyl oder
- für geradkettiges, verzweigtes oder cyclisches gesättigtes oder ungesättigtes, gegebenenfalls durch Hydroxy, Carboxy, Alkoxycarbonyl oder Halogen substituiertes Alkyl,

$R^3$ - für Wasserstoff oder
- für geradkettiges, verzweigtes oder cyclisches Alkyl oder Alkenyl steht, das gegebenenfalls substituiert ist durch Halogen, Cyano, Hydroxy, Amino, Alkylamino, Dialkylamino oder durch eine 5-7 gliedrigen heterocyclischen Ring der als Heteroatome Stickstoff und/oder Sauerstoff und/oder Schwefel enthalten kann und sowohl gesättigt als auch ungesättigt sein kann,

und

$R^5$ - für Wasserstoff,
- für Cyano,
- für Nitro steht,

oder

$R^4$ und $R^5$ gemeinsam einen Ring bilden wie

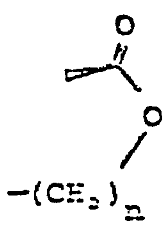 ,

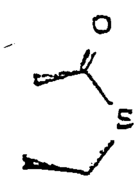 oder

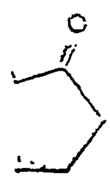

wobei n für 1 oder 2 steht,

und

b) Dihydropyridinen der allgemeinen Formel (II)

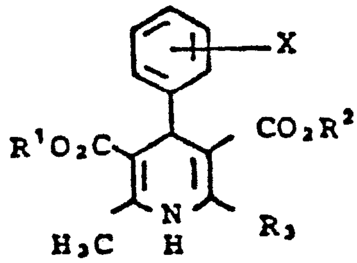

(II)

in welcher

$R^1$ - für gegebenenfalls durch $C_1$-$C_3$ Alkoxy substituiertes $C_1$-$C_4$ Alkyl steht,

$R^2$ - für $C_1$-$C_{10}$ Alkyl steht, das gegebenenfalls substituiert ist durch $C_1$-$C_3$ Alkoxy, Trifluormethyl, Trifluorethyl, N-Methyl-N-benzylamino,

$R^3$ - für Cyano,
- für Hydroxymethyl oder
- für $C_1$-$C_4$ Alkyl steht,

und

X - für 2-Nitro, 3-Nitro, 2-Chlor, 2,3-Dichlor, 2,3-Ringglied bestehend aus =N-O-N= steht,

dadurch gekennzeichnet, daß man die Komponenten a) und b) in einem inerten Lösungsmittel löst und diese Lösungen mischt.

11. Verfahren zur Herstellung der Mischung gemäß Anspruch 10, dadurch gekennzeichnet, daß als Komponente a) Dihydropyridine der allgemeinen Formel (I) in Anspruch 10, in welcher

R - für $C_4$-$C_{12}$ Cycloalkyl steht, oder
- für Phenyl, Naphthyl, Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Chinolyl, Isochinolyl, Indolyl, Benzimidazolyl, Chinazolyl, Chinoxalyl, Thionaphthenyl, Isothionaphthenyl, Chromonyl, Thiochromonyl, Chromenyl, Thiochromenyl, Benzoxadiazolyl oder Benzthiadiazolyl steht, wobei die genannten Reste gegebenenfalls substituiert sind durch bis zu vier gleiche oder verschiedene Substituenten aus der Reihe: Fluor, Chlor, Brom, Jod, Nitro, Cyan, Trifluormethyl, Monofluor-$C_1$-$C_8$-alkoxy, Polyfluor-$C_1$-$C_8$-alkoxy, Hydroxy, Amino, Alkylamino, Di-$C_1$-$C_6$-alkylamino, Phenyl, Naphthyl, Thienyl, Furyl, Pyridyl, Pyrimidyl, Benzyl, -O-Benzyl, -$SO_n$-Benzyl (n = 0-2) wobei die Aromaten und Heteroaromaten gegebenenfalls wiederum ein- bis vierfach substituiert sein können durch Fluor, Chlor, Brom, Jod, Cyano,Nitro, Azido, Hydroxy, Trifluormethyl, Trifluormethoxy, Amino, Alkylamino, Di-$C_1$-$C_6$-alkylamino, Alkoxy oder $C_1$-$C_2$-Alkylthio,

$R^1$ - für einen geradkettigen, verzweigten oder cyclisch gesättigten oder ungesättigten Kohlenwasserstoffrest mit bis zu 17 C-Atomen steht, der gegebenenfalls in der Kette durch bis zu vier Sauerstoff- und/oder Schwefelatome unterbrochen ist und der gegebenenfalls substituiert ist durch ein oder mehrere Fluor, Chlor, Brom, Jod, Nitro, Hydroxy, Cyano, Tri-$C_1$-$C_6$-alkylsilyl, $C_1$-$C_2$-Alkoxycarbonyl, Amino, Alkylamino oder Di-$C_1$-$C_2$-alkylamino,

$R^2$,$R^4$ - gleich oder verschieden sind und
- für Wasserstoff,
- für Amino,
- für Cyano,
- für Formyl oder
- für geradkettiges, verzweigtes oder cyclisches, gegebenenfalls durch Hydroxy, Carboxy, $C_1$-$C_2$-Alkoxycarbonyl, ein oder mehrere Fluor, Chlor oder Brom substituiertes $C_1$-$C_8$-Alkyl oder -Alkenyl stehen,

$R^3$ - für Wasserstoff oder
- für geradkettiges, verzweigtes oder cyclisches, $C_1$-$C_8$-Alkyl oder -Alkenyl steht, das gegebenenfalls durch bis zu 3 Sauerstoffatome in der Kette unterbrochen ist und das gegebenenfalls substituiert ist durch ein oder mehrere Fluor, Chlor, Brom, Jod, Cyano, Hydroxy, Amino, $C_1$-$C_2$-Alkyl, Di-$C_1$-$C_2$-alkylamino, Morpholin, Piperidin, Pyridazin oder Pyridin,

und

$R^5$ - für Wasserstoff,
- für Cyano,
- für Nitro steht,

oder

$R^4$ und $R^5$ gemeinsam einen Ring bilden wie

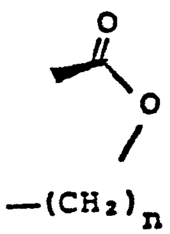
,
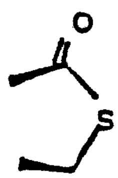
oder
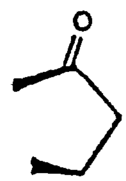

wobei n für 1 oder 2 steht,

eingesetzt werden.

12. Verfahren zur Herstellung der Mischung gemäß Anspruch 10, dadurch gekennzeichnet, daß als Komponente a) Dihydropyridine der allgemeinen Formel (I) in Anspruch 10, in welcher

R - für $C_5$-$C_{10}$-Cycloalkyl steht,
- für Phenyl, Naphthyl, Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Chinazolyl, Chinoxalyl, Thionaphthenyl, Isothionaphthenyl, Chromoyl, Thiochromonyl, Chromenyl, Thiochromenyl, Benzoxadiazolyl oder Benzthiadiazolyl steht,

 0186027

wobei die genannten Reste gegebenenfalls substituiert sind durch bis zu drei gleiche oder verschiedene Substituenten der Reihe: Fluor, Chlor, Brom, Nitro, Cyano, Trifluormethyl, Monofluor-$C_1$-$C_4$-alkoxy, Polyfluor-$C_1$-$C_4$-alkoxy, Hydroxy, Amino, $C_1$-$C_4$-Alkylamino, Di-$C_1$-$C_4$-alkylamino, Phenyl, Thienyl, Pyridyl, Benzyl, -O-Benzyl, -$SO_n$-Benzyl (n = 0 bis 2), wobei die Aromaten und Heteroaromaten gegebenenfalls wiederum ein- bis dreifach substituiert sein können durch Fluor, Chlor, Brom, Cyano, Nitro, Hydroxy, Trifluormethyl, Trifluormethoxy, Amino, $C_1$-$C_4$-Alkylamino, Di-$C_1$-$C_4$-alkylamino, Methoxy oder Methylthio,

$R^1$ - für einen geradkettigen, verzweigten oder cyclischen, gesättigten oder ungesättigten Kohlenwasserstoffrest mit bis zu 14 C-Atomen

steht, der gegebenenfalls durch bis zu 3 Sauerstoff- und/oder Schwefelatome in der Kette unterbrochen sein kann und der gegebenenfalls substituiert ist durch ein oder mehrere Fluor, Chlor, Brom, Nitro, Hydroxy, Cyano, oder Tri-$C_1$-$C_4$-alkylsilyl,

$R^2$,$R^4$ - gleich oder verschieden sind und
- für Wasserstoff,
- für Amino,
- für Cyano,
- für Formyl oder
- für geradkettiges oder verzweigtes, gegebenenfalls durch Hydroxy substituiertes $C_1$-$C_4$-Alkyl oder -Alkenyl stehen,

$R^3$ - für Wasserstoff oder

- für geradkettiges, verzweigtes oder cyclisches $C_1$-$C_6$-Alkyl oder -Alkenyl steht, das gegebenenfalls durch bis zu zwei Sauerstoffatome in der Kette unterbrochen ist und das gegebenenfalls substituiert ist durch ein oder mehrere Fluor, Chlor, Cyan, Hydroxy, Amino oder Morpholino,

und

$R^5$ - für Wasserstoff,

- für Cyano,
- für Nitro steht,

oder

$R^4$ und $R^5$ gemeinsam einen Ring bilden wie

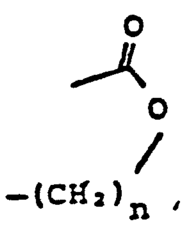

,

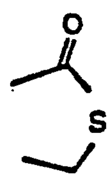

oder

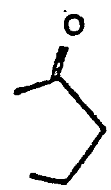

wobei n für 1 oder 2 steht,

eingesetzt werden.

0186027

13. Verfahren zur Herstellung der Mischung gemäß Anspruch 10, dadurch gekennzeichnet, daß als Komponente a) 1,4-Dihydro-2,6-dimethyl-5-nitro-4-(2-trifluormethylphenyl)-pyridin-3-carbonsäuremethylester und/oder 2-Methyl-4-(4-oxo-2-phenyl-4H-thiochromen-8-yl)-5-oxo-1,4,5,7-tetrahydrofuro[3,4-b]pyridin-3-carbonsäureethylester eingesetzt werden.

14. Verfahren zur Herstellung der Mischung gemäß Anspruch 10, dadurch gekennzeichnet, daß als Komponente b) ein Dihydropyridin der Formel (II), in Anspruch 10, in welcher

X eine Nitrogruppe in 2- bzw. 3-Position, 2-Chlor, 2,3-Dichlor, oder eine 2,3 =N-O-N=Gruppe darstellt,

$R^1$ Methyl, Ethyl, Isopropyl, n-Propyloxyethyl darstellt,

$R^2$ Methyl, Ethyl, Isopropyl, Isobutyl, n-Decyl, n-Propyloxyethyl, Methoxyethyl, Trifluormethylethyl sowie die Gruppe $C_6H_5CH_2N(CH_3)-CH_2-CH_2-$ darstellt und

$R^3$ Methyl, Cyano und Hydroxymethyl darstellt,

eingesetzt wird.

15. Verfahren zur Herstellung der Mischung gemäß Anspruch 10, dadurch gekennzeichnet, daß als Komponente b) Nitrendipin, Nicardipin, Nisoldipin, Felodipin, Nifedipin, Nimodipin, PN 200-100, Dazodipin.

eingesetzt werden.

16. Verfahren zur Herstellung eines Arzneimittels enthaltend als Wirkstoffkombination eine Mischung aus

a) Dihydropyridinen der allgemeinen Formel (I)

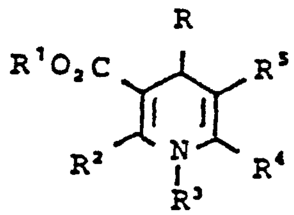

(I)

in welcher

R - für Cycloalkyl steht, oder
- Aryl oder Heteroaryl steht, die gegebenenfalls substituiert sind durch bis zu fünf gleiche oder verschiedene Substituenten der Reihe: Halogen, Nitro, Cyano, Trifluormethyl, Monofluoralkoxy, Polyfluoralkoxy, Hydroxy, Amino, Alkylamino, Dialkylamino, Aryl, Heteroaryl, Aralkyl, -O-Aralkyl, oder $-SO_n$-Aralkyl (n = 0-2), wobei Substituenten der fünf letztgenannten Gruppen ebenfalls mehrfach substituiert sein können durch bis zu fünf Substituenten der Reihe: Halogen, Nitro, Azido, Hydroxy, Trifluormethyl, Trifluormethoxy, Cyano, Amino, Alkylamino, Dialkylamino, Alkoxy, Alkylthio,

$R^1$ - für einen geradkettigen, verzweigten oder cyclischen, gesättigten oder ungesättigten Kohlenwasserstoffrest steht, der gegebenenfalls durch

bis zu fünf Schwefel- und/oder Sauerstoffatome in der Kette unterbrochen ist und der gegebenenfalls substituiert ist durch Halogen, Nitro, Hydroxy, Cyano, Trialkylsilyl, Alkoxycarbonyl, Amino, Alkylamino oder Dialkylamino,

$R^2$, $R^4$ - gleich oder verschieden sind und
- für Wasserstoff,
- für Amino,
- für Cyano,
- für Formyl oder
- für geradkettiges, verzweigtes oder cyclisches gesättigtes oder ungesättigtes, gegebenenfalls durch Hydroxy, Carboxy, Alkoxycarbonyl oder Halogen substituiertes Alkyl,

$R^3$ - für Wasserstoff oder
- für geradkettiges, verzweigtes oder cyclisches Alkyl oder Alkenyl steht, das gegebenenfalls substituiert ist durch Halogen, Cyano, Hydroxy, Amino, Alkylamino, Dialkylamino oder durch eine 5-7 gliedrigen heterocyclischen Ring der als Heteroatome Stickstoff und/oder Sauerstoff und/oder Schwefel enthalten kann und sowohl gesättigt als auch ungesättigt sein kann,

und

$R^5$ - für Wasserstoff,
- für Cyano,
- für Nitro steht,

oder

$R^4$ und $R^5$ gemeinsam einen Ring bilden wie

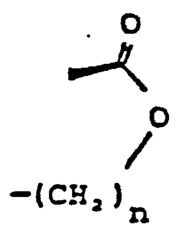 , 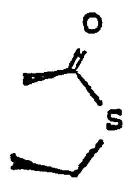 oder 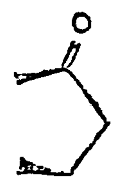

wobei n für 1 oder 2 steht,

und

b) Dihydropyridine der allgemeinen Formel (II)

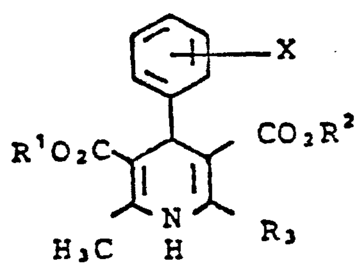

(II)

in welcher

$R^1$ - für gegebenenfalls durch $C_1$-$C_3$ Alkoxy substituiertes $C_1$-$C_4$ Alkyl steht,

$R^2$ - für $C_1$-$C_{10}$ Alkyl steht, das gegebenenfalls substituiert ist durch $C_1$-$C_3$ Alkoxy, Trifluormethyl, Trifluorethyl, N-Methyl-N-benzylamino,

$R^3$ - für Cyano,
- für Hydroxymethyl oder
- für $C_1$-$C_4$ Alkyl steht,

und

X - für 2-Nitro, 3-Nitro, 2-Chlor, 2,3-Dichlor, 2,3-Ringglied bestehend aus =N-O-N= steht,

dadurch gekennzeichnet, daß man die Komponenten a) und b) in einem inerten Lösungsmittel löst, anschließend die Lösungen mischt und übliche Hilfs- und Trägerstoffe zufügt.

17. Verwendung der Mischung gemäß Ansprüche 1 bis 7 zur Bekämpfung von Kreislauferkrankungen.

18. Verwendung der Mischung gemäß Ansprüche 1 bis 7 zur Herstellung von Arzneimitteln zur Bekämpfung von Herz-Kreislauferkrankungen.